(19) Europäisches Patentamt
European Patent Office
Office européen des brevets

(11) Veröffentlichungsnummer: 0 410 552 A1

# (12) EUROPÄISCHE PATENTANMELDUNG

(21) Anmeldenummer: 90250189.9

(22) Anmeldetag: 25.07.90

(51) Int. Cl.5: C07D 417/06, A01N 43/56, C07D 413/06, C07D 403/06, C07D 231/20, A01N 43/78, A01N 43/82, A01N 43/76, A01N 43/50, A01N 43/80

(30) Priorität: 28.07.89 DE 3925502

(43) Veröffentlichungstag der Anmeldung: 30.01.91 Patentblatt 91/05

(84) Benannte Vertragsstaaten: AT BE CH DE DK ES FR GB GR IT LI LU NL SE

(71) Anmelder: SCHERING AKTIENGESELLSCHAFT
Berlin und Bergkamen
Müllerstrasse 170/178 Postfach 65 03 11
D-1000 Berlin 65(DE)

(72) Erfinder: Angermann, Alfred, Dr.
Nussbaumallee 12
D-1000 Berlin 19(DE)
Erfinder: Franke, Helga, Dr.
Spiessergasse 6b
D-1000 Berlin 27(DE)
Erfinder: Geisler, Jens, Dr.
Schwendyweg 13
D-1000 Berlin 20(DE)
Erfinder: Johann, Gerhard, Dr.
Hermsdorfer Damm 147
D-1000 Berlin 28(DE)
Erfinder: Rees, Richard, Dr.
Speerweg 8
D-1000 Berlin 28(DE)

## (54) Substituierte 4-Heteroaroylpyrazole, Verfahren zur Herstellung dieser Verbindungen und ihre Verwendung als Mittel mit herbizider Wirkung.

(57) Die Erfindung betrifft neue substituierte 4-Heteroaroylpyrazole der allgemeinen Formel I

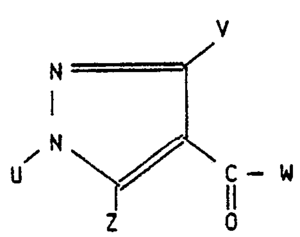

(I) ,

in der U, V, W und Z die in der Beschreibung genannten Bedeutungen haben, Verfahren zu ihrer Herstellung und ihre Verwendung als Mittel mit herbizider Wirkung.

EP 0 410 552 A1

# SUBSTITUIERTE 4-HETEROAROYLPYRAZOLE, VERFAHREN ZUR HERSTELLUNG DIESER VERBINDUNGEN UND IHRE VERWENDUNG ALS MITTEL MIT HERBIZIDER WIRKUNG

Die Erfindung betrifft neue substituierte 4-Heteroaroylpyrazole, Verfahren zur Herstellung dieser Verbindungen und ihre Verwendung als Mittel mit herbizider Wirkung.

Es ist bereits bekannt, daß gewisse 4-Benzoyl- und 4-Pyridylcarbonylpyrazolderivate eine herbizide Wirkung besitzen (JP-A 51/106 738 und JP-A 54/009 279). Häufig ist die herbizide Wirkung dieser bekannten Verbindungen aber nicht ausreichend, oder es treten Selektivitätsprobleme in wichtigen landwirtschaftlichen Kulturen auf.

Aufgabe der vorliegenden Erfindung ist daher die Bereitstellung von neuen Verbindungen, die in ihren biologischen Eigenschaften den bisher bekannten Verbindungen überlegen sind.

Diese Aufgabe wird gelöst durch neue substituierte 4-Heteroaroylpyrazole der allgemeinen Formel I

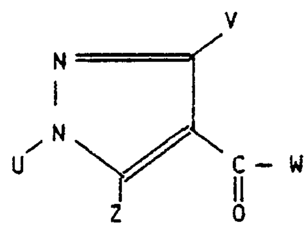

(I) ,

in der

U ein Wasserstoffatom, einen $C_1$-$C_4$-Alkylrest oder einen Halogen-$C_1$-$C_4$-alkylrest,
V ein Wasserstoffatom, einen $C_1$-$C_4$-Alkylrest oder einen Halogen-$C_1$-$C_4$-alkylrest,
W eine heterocyclische Gruppe W-1 bis W-7 der allgemeinen Formeln

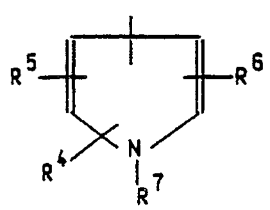

W - 1

,

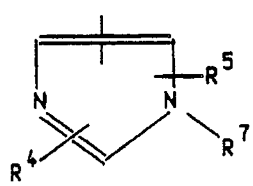

W - 2

,

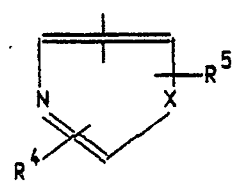

W - 3

,

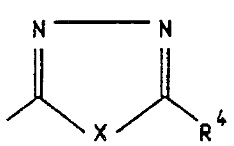

W - 4

,

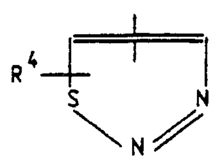

W - 5

,

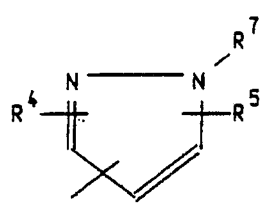

W - 6

oder

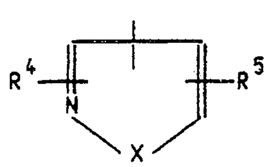

W - 7

X ein Sauerstoffatom oder ein Schwefelatom,

Z ein Halogenatom oder eine der Gruppen $-OR^1$, $-O-(CH_2)_n-CO-R^1$, $-O-SO_2-R^1$, $-S(O)_n-R^1$, -OM oder $-NR^2R^3$,

n 0, 1 oder 2,

M ein Kation aus der Gruppe Lithium, Natrium und Kalium, ein Äquivalent aus der Gruppe Zink, Mangan, Calcium, Magnesium und Barium oder ein Ammoniumion der allgemeinen Formel

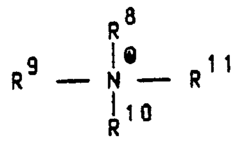

,

$R^1$ ein Wasserstoffatom, einen $C_1$-$C_{18}$-Alkylrest, einen ein- oder mehrfach, gleich oder verschieden durch Halogen, Hydroxy oder Cyano substituierten oder durch Sauerstoff oder Schwefel ein- oder mehrfach unterbrochenen $C_1$-$C_{18}$-Alkylrest, einen $C_2$-$C_{12}$-Alkenylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkinylrest, einen Phenylrest, einen ein- oder mehrfach, gleich oder verschieden durch Halogen, Hitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenyl rest, einen Phenyl-$C_1$-$C_3$-alkylrest oder einen ein-oder mehrfach, gleich oder verschieden durch Halogen, Nitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenyl-$C_1$-$C_3$-alkylrest,

$R^2$ ein Wasserstoffatom, einen $C_1$-$C_{18}$-Alkylrest, einen ein- oder mehrfach, gleich oder verschieden durch Halogen, Hydroxy, Cyano, $C_1$-$C_4$-Alkoxycarbonyl, einen Rest $-NR^{12}R^{13}$ oder einen Rest $-N^{\oplus}R^{12}R^{13}R^{14}$ substituierten oder durch Sauerstoff oder Schwefel ein- oder mehrfach unterbrochenen $C_1$-$C_{18}$-Alkylrest, einen $C_2$-$C_{12}$-Alkenylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkinylrest, einen $C_1$-$C_6$-Alkoxyrest, einen Halogen-$C_1$-$C_6$-alkoxyrest, einen Phenylrest, einen ein- oder mehrfach, gleich oder verschieden durch Halogen, Nitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenylrest, einen Phenyl-$C_1$-$C_3$-alkylrest oder einen ein- oder mehrfach, gleich oder verschieden durch Halogen, Nitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenyl-$C_1$-$C_3$-alkylrest,

$R^3$ ein Wasserstoffatom, einen $C_1$-$C_{18}$-Alkylrest, einen ein- oder mehrfach, gleich oder verschieden durch Halogen, Hydroxy, Cyano, $C_1$-$C_4$-Alkoxycarbonyl, einen Rest $-NR^{12}R^{13}$ oder einen Rest $-N^{\oplus}R^{12}R^{13}R^{14}$ substituierten oder durch Sauerstoff oder Schwefel ein- oder mehrfach unterbrochenen $C_1$-$C_{18}$-Alkylrest einen $C_2$-$C_{12}$-Alkenylrest, einen ein-oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein-oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkinylrest, einen Phenylrest, einen ein- oder mehrfach, gleich oder verschieden durch Halogen, Nitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenylrest, einen Phenyl-$C_1$-$C_3$-alkylrest oder einen ein- oder mehrfach, gleich oder verschieden durch Halogen, Nitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenyl-$C_1$-$C_3$-alkylrest oder

$R^2$ und $R^3$ gemeinsam mit dem benachbarten Stickstoffatom eine Morpholinogruppe, eine Piperidinogruppe oder eine Pyrrolidinogruppe,

$R^4$ ein Wasserstoffatom, ein Halogenatom, einen $C_1$-$C_{12}$-Alkylrest, einen durch Sauerstoff oder Schwefel ein- oder mehrfach unterbrochenen $C_2$-$C_{12}$-Alkylrest, einen Halogen-$C_1$-$C_4$-alkylrest, einen $C_1$-$C_4$-Alkoxyrest, einen Halogen-$C_1$-$C_4$-alkoxyrest, einen $C_1$-$C_4$-Alkylmercaptorest, einen $C_1$-$C_4$-Alkoxycarbonylrest, einen Phenylrest, einen durch Halogen, Nitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenylrest, eine Cyanogruppe oder eine Nitrogruppe,

$R^5$ ein Wasserstoffatom, ein Halogenatom, einen $C_1$-$C_{12}$-Alkylrest, einen durch Sauerstoff oder Schwefel ein- oder mehrfach unterbrochenen $C_2$-$C_{12}$-Alkylrest, einen Halogen-$C_1$-$C_4$-alkylrest, einen $C_1$-$C_4$-Alkoxyrest, einen Halogen-$C_1$-$C_4$-alkoxyrest, einen $C_1$-$C_4$-Alkylmercaptorest, einen $C_1$-$C_4$-Alkoxycarbonylrest, einen Phenylrest, einen durch Halogen, Nitro, Cyano, $C_1$-$C_4$-Alkyl, Halogen-$C_1$-$C_4$-alkyl, $C_1$-$C_4$-Alkoxy oder

Halogen-$C_1$-$C_4$-alkoxy substituierten Phenylrest, eine Cyanogruppe oder eine Nitrogruppe,

$R^6$ ein Wasserstoffatom, ein Halogenatom, einen $C_1$-$C_{12}$-Alkylrest, einen durch Sauerstoff oder Schwefel ein- oder mehrfach unterbrochenen $C_2$-$C_{12}$-Alkylrest, einen Halogen-$C_1$-$C_4$-alkylrest, einen $C_1$-$C_4$-Alkoxyrest, einen Halogen-$C_1$-$C_4$-alkoxyrest, einen $C_1$-$C_4$-Alkylmercaptorest, einen $C_1$-$C_4$-Alkoxycarbonylrest, einen Phenylrest, einen durch Halogen, Nitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenylrest, eine Cyanogruppe oder eine Nitrogruppe,

$R^7$ ein Wasserstoffatom, ein Halogenatom, einen $C_1$-$C_{12}$-Alkylrest, einen durch Sauerstoff oder Schwefel ein- oder mehrfach unterbrochenen $C_2$-$C_{12}$-Alkylrest, einen Halogen-$C_1$-$C_4$-alkylrest, einen $C_1$-$C_4$-Alkoxyrest, einen Halogen-$C_1$-$C_4$-alkoxyrest, einen $C_1$-$C_4$-Alkylmercaptorest, einen $C_1$-$C_4$-Alkoxycarbonylrest, einen Phenylrest, einen durch Halogen, Nitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenylrest, eine Cyanogruppe oder eine Nitrogruppe,

$R^8$ ein Wasserstoffatom, einen $C_1$-$C_{12}$-Alkylrest, einen durch Sauerstoff oder Schwefel ein- oder mehrfach unterbrochenen $C_2$-$C_{12}$-Alkylrest, einen Phenylrest oder einen Benzylrest,

$R^9$ ein Wasserstoffatom, einen $C_1$-$C_{12}$-Alkylrest, einen durch Sauerstoff oder Schwefel ein- oder mehrfach unterbrochenen $C_2$-$C_{12}$-Alkylrest, einen Phenylrest oder einen Benzylrest,

$R^{10}$ ein Wasserstoffatom, einen $C_1$-$C_{12}$-Alkylrest, einen durch Sauerstoff oder Schwefel ein- oder mehrfach unterbrochenen $C_2$-$C_{12}$-Alkylrest, einen Phenylrest oder einen Benzylrest,

$R^{11}$ ein Wasserstoffatom, einen $C_1$-$C_{12}$-Alkylrest, einen durch Sauerstoff oder Schwefel ein- oder mehrfach unterbrochenen $C_2$-$C_{12}$-Alkylrest, einen Phenylrest oder einen Benzylrest,

$R^{12}$ ein Wasserstoffatom oder einen $C_1$-$C_{12}$-Alkylrest,

$R^{13}$ ein Wasserstoffatom oder einen $C_1$-$C_{12}$-Alkylrest und

$R^{14}$ ein Wasserstoffatom oder einen $C_1$-$C_{12}$-Alkylrest bedeuten.

Die Bezeichnung "Halogen" umfaßt Flucr, Chlor, Brom und Jod. "Halogenalkyl" oder "Halogenalkoxy" bedeutet, daß ein oder mehrere Wasserstoffatome durch Fluor, Chlor, Brom oder Jod ersetzt sind.

Unter den Begriffen "Alkyl", "Alkenyl" und "Alkinyl" sind sowohl geradkettige als auch verzweigte Kohlenwasserstoffreste zu verstehen.

Die erfindungsgemäßen Verbindungen der allgemeinen Formel I können, wenn Z Hydroxy bedeutet, auch als Tautomere mit den allgemeinen Formeln I′ und I″ vorliegen,

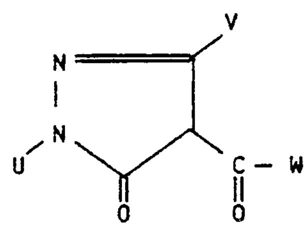

I'

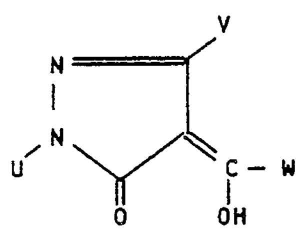

I''

in denen U, V und W die unter der allgemeinen Formel I angegebene Bedeutung besitzen.

Solche Strukturen werden ebenfalls durch die vorliegende Erfindung umfaßt, aus Vereinfachungsgründen wird aber jeweils nur die Struktur der allgemeinen Formel I angegeben.

Die Herstellung der erfindungsgemäßen Verbindungen kann in Anlehnung an bekannte Methoden durchgeführt werden (zum Beispiel Acta Chem. Scand. 13 , 1668 (1959); J. Med. Chem. 24 , 982 (1981); EP 0282944), beispielsweise indem man

A) falls Z eine Hydroxygruppe bedeutet, eine Verbindung der allgemeinen Formel II

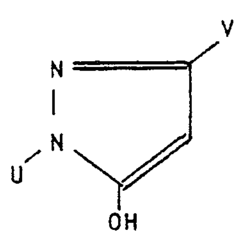

(II) ,

in der U und V die unter der allgemeinen Formel I genannten Bedeutungen haben, mit einem

Carbonsäurederivat der allgemeinen Formel III

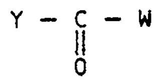

(III) ,

in der W die unter der allgemeinen Formel I genannten Bedeutungen hat und Y für ein Halogenatom, vorzugsweise ein Chloratom oder ein Bromatom, oder einen $C_1$-$C_4$-Alkoxyrest steht, in Anwesenheit einer geeigneten Base oder einer Lewis-Säure zur Reaktion bringt oder

B) falls Z eine Hydroxygruppe bedeutet, eine Verbindung der allgemeinen Formel II

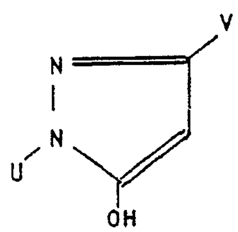

(II) ,

in der U und V die unter der allgemeinen Formel I genannten Bedeutungen haben, mit einer Carbonsäure der allgemeinen Formel IV

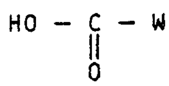

(IV) ,

in der W die unter der allgemeinen Formel I genannten Bedeutungen hat, in Anwesenheit eines wasserentziehenden Mittels und einer geeigneten Base oder einer Lewis-Säure zur Reaktion bringt oder

C) falls Z für die Gruppe $-OR^1$ steht, eine Verbindung der allgemeinen Formel V

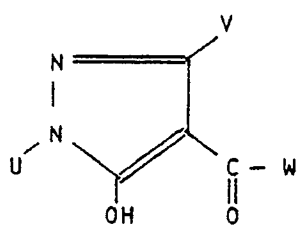

(V) ,

in der U, V und W die unter der allgemeinen Formel I genannten Bedeutungen haben, mit einem Alkylierungsmittel der allgemeinen Formel VI

$R^1$ - Y (VI) ,

in der $R^1$ die unter der allgemeinen Formel I genannten Bedeutungen hat und Y für die unter der allgemeinen Formel III angegebenen Bedeutungen steht, umsetzt oder

D) falls Z für die Gruppe $-O-(CH_2)_n-CO-R^1$ steht, eine Verbindung der allgemeinen Formel V

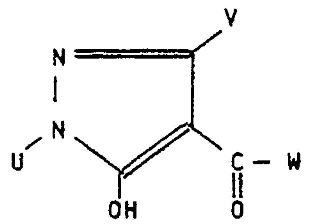

(V) ,

in der U, V und W die unter der allgemeinen Formel I genannten Bedeutungen haben, mit einem Alkylierungsmittel oder einem Acylierungsmittel der allgemeinen Formel VII

$R^1$-CO-$(CH_2)_n$-Y (VII) ,

in der $R^1$ und n die unter der allgemeinen Formel I genannten Bedeutungen haben und Y für die unter der allgemeinen Formel III angegebenen Bedeutungen steht, umsetzt oder

E) falls Z für die Gruppe -O-$SO_2$-$R^1$ steht, eine Verbindung der allgemeinen Formel V

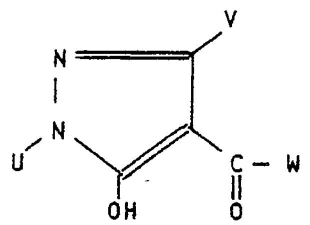

(V) ,

in der U, V und W die unter der allgemeinen Formel I genannten Bedeutungen haben, mit einem Sulfonylierungsmittel der allgemeinen Formel VIII

$R^1$-$SO_2$-Y (VIII) ,

in der $R^1$ die unter der allgemeinen Formel I genannten Bedeutungen hat und Y für die unter der allgemeinen Formel III angegebenen Bedeutungen steht, umsetzt oder

F) falls Z für die Gruppe -OM steht, eine Verbindung der allgemeinen Formel V

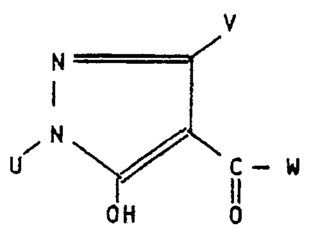

(V) ,

in der U, V und W die unter der allgemeinen Formel I genannten Bedeutungen haben, mit einer Base der allgemeinen Formel IX

M - Q (IX) ,

in der M die unter der allgemeinen Formel I angegebenen Bedeutungen hat und Q für eine Hydroxygruppe oder ein Carbonatäquivalent steht, umsetzt oder

G) falls Z für ein Halogenatom steht, eine Verbindung der allgemeinen Formel V

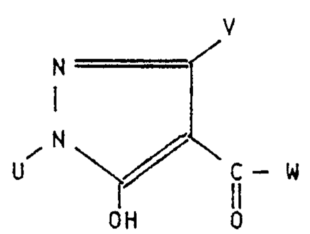

(V) ,

in der U, V und W die unter der allgemeinen Formel I genannten Bedeutungen haben, nach an sich bekannten Verfahren halogeniert oder

H) falls Z für die Gruppe $-NR^2R^3$ steht, eine Verbindung der allgemeinen Formel X

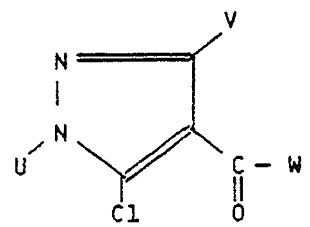

(X) ,

in der U, V und W die unter der allgemeinen Formel I genannten Bedeutengen haben, mit einem Amin der allgemeinen Formel XI

$HNR^2R^3$ (XI) ,

in der $R^2$ und $R^3$ die unter der allgemeinen Formel I genannten Bedeutungen haben, umsetzt oder

I) falls 2 für die Grupppe $-S(O)_n-R^1$ steht, eine Verbindung der allgemeinen Formel X

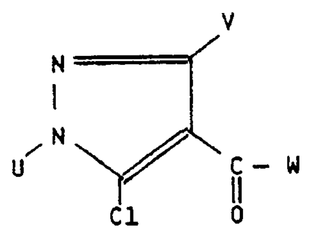

(X) ,

in der U, V und W die unter der allgemeinen Formel I genannten Bedeutungen haben, mit einem Thioalkohol der allgemeinen Formel XII

$HSR^1$ (XII),

in der $R^1$ die unter der allgemeinen Formel I genannten Bedeutungen hat, umsetzt und das Reaktionsprodukt gewünschtenfalls anschließend oxydiert.

Als Basen für die Varianten A) und B) eignen sich Pyridinderivate, wie zum Beispiel 4-(N,N-Dialkylamino)-pyridine, vorzugsweise 4-(N,N-Dimethylamino)-pyridin, N-Alkylimidazole, wie zum Beispiel N-Methylimidazol, Metallcyanide, wie zum Beispiel Kupfer-1-cyanid, Natriumcyanid, Kaliumcyanid und Zinkcyanid, 2-Hydroxyisobutansäurenitril, Alkali- und Erdalkalicarbonate und -hydroxyde, wie zum Beispiel Kalium- und Natriumcarbonat oder Kalium-, Natrium-, Magnesium- und Calciumhydroxyd, sowie Alkoholate, wie zum Beispiel Kalium-, Natrium-, Calcium- oder Magnesiumethylat und -methylat.

Als Lewis-Säuren können bei den Varianten A) und B) unter anderem Zink-, Aluminium- und Borhalogenide, beispielsweise Zink- oder Aluminiumchlorid und -bromid oder Bortrifluorid zum Einsatz kommen.

Als wasserentziehendes Mittel bei Variante B) hat sich unter anderem N,N-Dicyclohexylcarbodimid als besonders vorteilhaft erwiesen.

Die Variante G) kann beispielsweie so durchgefühert werden, daß man die nach A) oder B) erhaltene Hydroxyverbindung in einem inerten Lösungsmittel, vorzugsweise einem chlorierten Kohlenwasserstoff, löst und mit einem Überschuß an Oxalylchlorid sowie katalytischen Mengen Dimethylformamid behandelt.

Die Herstellungsmethoden A) bis I) werden vorzugsweise in Anwesenheit eines geeigneten Lösungs- und bzw. Verdünnungsmittels durchgeführt.

Dabei können jeweils alle solchen Lösungs- beziehungsweise Verdünnungsmittel zum Einsatz kommen, die gegenüber den jeweiligen Reaktanden inert sind. Beispiele für solche Lösungsmittel beziehungsweise Verdünnungsmittel sind aliphatische, alicyclische und aromatische Kohlenwasserstoffe, die jeweils gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylenchlorid, Trichlorethylen und Chlorbenzol, Ether, wie zum Beispiel Diethylether, Methylethylether, Methyl-t-butylether, Diisopropylether, Dibutylether, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum Beispiel Methanol, Ethanol, Isopropanol, Butanol, tert.-Butanol, tert.-Amylalkohol und Ethylenglycol, Ester, wie zum

Beispiel Ethylacetat und Amylacetat, Säureamide, wie zum Beispiel Dimethylformamid und Dimethylacetamid, Sulfoxide, wie zum Beispiel Dimethylsulfoxid, und Sulfone, wie zum Beispiel Sulfolan, sowie Basen, wie zum Beispiel Pyridin und Triethylamin.

Alle Reaktionen werden vorzugsweise unter dem Druck der Umgebung durchgeführt, wenngleich sie auch bei erhöhtem oder vermindertem Druck ausgeführt werden könnten. Sie können ferner innerhalb eines breiten Temperaturbereichs durchgeführt werden. Im allgemeinen werden sie bei einer Temperatur zwischen -20°C und dem Siedepunkt des Reaktionsgemisches, vorzugsweise bei 0°C bis 150°C, durchgeführt.

Die Gegenwart eines zusätzlichen Reaktionskatalysators kann von Vorteil sein. Als solche Katalysatoren sind Kaliumjodid und Oniumverbindungen, wie quarternäre Ammonium-, Phosphonium-, Arsonium- und Sulfoniumverbindungen, geeignet. Ebenfalls geeignet sind Polyglycolether, insbesondere cyclische, wie zum Beispiel 18-Krone-6, und tertiäre Amine, wie zum Beispiel Tributylamin. Bevorzugt sind quarternäre Ammoniumverbindungen, wie zum Beispiel Benzyltriethylammoniumbromid und Tetrabutylammoniumbromid.

Die nach den oben genannten Verfahren hergestellten erfindungsgemäßen Verbindungen können nach den üblichen Methoden aus dem Reaktionsgemisch isoliert werden, beispielsweise durch Abdestillieren des eingesetzten Lösungsmittels bei normalem oder vermindertem Druck, durch Ausfällen mit Wasser oder durch Extraktion. Ein erhöhter Reinheitsgrad kann in der Regel durch säulenchromatographische Aufreinigung sowie durch fraktionierte Destillation oder Kristallisation erhalten werden.

Die erfindungsgemäßen Verbindungen stellen in der Regel fast farb- und ge ruchlose Flüssigkeiten oder Kristalle dar, die bedingt löslich in Wasser, aliphatischen Kohlenwasserstoffen, wie Petrolether, Hexan, Pentan und Cyclohexan, gut löslich in halogenierten Kohlenwasserstoffen, wie Chloroform, Methylenchlorid und Tetrachlorkohlenstoff, aromatischen Kohlenwasserstoffen, wie Benzol, Toluol und Xylol, Ethern, wie Diethylether, Tetrahydrofuran und Dioxan, Carbonsäurenitrilen, wie Acetonitril, Alkoholen, wie Methanol und Ethanol, Carbonsäureamiden, wie Dimethylformamid, Sulfoxiden, wie Dimethylsulfoxid, und - falls Z eine Hydroxygruppe bedeutet - auch in Basen wie Alkalihydroxid-, Alkalicarbonat- und Alkalihydrogencarbonatlösungen, Triethylamin oder Pyridin sind.

Die bei den Herstellungsverfahren A) und B) zur Acylierung benötigten Verbindungen der allgemeinen Formeln III und IV sind entweder literaturbekannt oder können in Anlehung an literaturbekannte Methoden hergestellt werden. Einschlägige Techniken sind beispielsweise in J. Am. Chem. Soc. 77 , 5359 (1955) oder Chem. Ber. 99 , 1618 (1966) beschrieben.

Die erfindungsgemäßen Verbindungen zeigen eine gute herbizide Wirkung bei breitblättrigen Unkräutern und Gräsern. Ein selektiver Einsatz der erfindungsgemäßen Wirkstoffe ist in verschiedenen Kulturen möglich, zum Beispiel in Raps, Rüben, Sojabohnen, Baumwolle, Reis, Gerste, Weizen und anderen Getreidearten. Dabei sind einzelne Wirkstoffe als Selektivherbizide in Rüben, Baumwolle, Soja und Getreide besonders geeignet. Ebenso können die Verbindungen zur Unkrautbekämpfung in Dauerkulturen, wie zum Beispiel Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, und zur selektiven Unkrautbekämpfung in einjährigen Kulturen eingesetzt werden.

Die erfindungsgemäßen Verbindungen können zum Beispiel bei den folgenden Pflanzengattungen verwendet werden:

Dikotyle Unkräuter der Gattungen Sinapis, Lepidium, Galium, Stellaria, Matricaria, Anthemis, Galinsoga, Chenopodium, Brassica, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Lamium, Veronica, Abutilon, Datura, Viola, Galeopsis, Papaver, Centaurea und Chrysanthemum.

Monokotyle Unkräuter der Gattungen Avena, Alopecurus, Echinochloa, Setaria, Panicum, Digitaria, Poa, Eleusine, Brachiaria, Lolium, Bromus, Cyperus, Agropyron, Sagittaria, Monochoria, Fimbristylis, Eleocharis, Ischaemum und Apera.

Die Aufwandmengen schwanken je nach Anwendungsart im Vor- und Nachauflauf in Grenzen zwischen 0,001 bis 5 kg/ha.

Die erfindungsgemäßen Wirkstoffe können auch als Defoliant, Desiccant und als Krautabtötungsmittel verwendet werden.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden. Sofern eine Verbreiterung des Wirkungsspektrums beabsichtigt ist, können auch andere Herbizide zugesetzt werden.

Beispielsweise eignen sich als herbizid wirksame Mischungspartner diejenigen Wirkstoffe, die in Weed Abstracts, Vol. 38, No. 3, 1989, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Zweckmäßig werden die gekennzeichneten Wirkstoffe oder deren Mischungen in Form von Zübereitungen wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

A) **Spritzpulver**

1.) 20 Gewichtsprozent Wirkstoff
68 Gewichtsprozent Kaolin
10 Gewichtsprozent Calciumsalz der Ligninsulfonsäure
2 Gewichtsprozent Dialkylnaphthalinsulfonat

2.) 40 Gewichtsprozent Wirkstoff
25 Gewichtsprozent Kaolin
25 Gewichtsprozent kolloidale Kieselsäure
8 Gewichtsprozent Calciumsalz der Ligninsulfonsäure
2 Gewichtsprozent Natriumsalz des N-Methyl-M-oleyl-taurins

B) **Emulsionskonzentrat**

20 Gewichtsprozent Wirkstoff
75 Gewichtsprozent Isophoron
2 Gewichtsprozent ethoxyliertes Rizinusöl
3 Gewichtsprozent Calciumsalz der Dodecylphenylsulfonsäure

Die folgenden Beispiele erläutern die Herstellung der erfindungsgemäßen Verbindungen:

**Beispiel 1.01**

5-Hydroxy-1,3-dimethyl-4-(4-methyl-1,2,3-thiadiazol-5-ylcarbonyl)-pyrazol

2,24 g (20 mmol) 5-Hydroxy-1,3-dimethylpyrazol, 2,88 g (20 mmol) 4-Methyl-1,2,3-thiadiazol-5-carbonsäure, 4,13 g (20 mmol) N,N-Dicyclohexylcarbodiimid und 1,38 g (10 mmol) Kaliumcarbonat wurden in 60 ml 2-Methylbutan-2-ol 3 Stunden auf 80°C erhitzt. Nach Abdestillieren des Lösungsmittels wurde in Wasser aufgenommen, abgesaugt und das Filtrat zweimal mit Chloroform gewaschen. Ansäuern der wäßrigen Phase mit konzentrierter Salzsäure bis pH 2 und Extraktion mit Chloroform lieferte nach Trocknen (Magnesiumsulfat), Einrotieren und Umkristallisation die Zielverbindung in Form gelblicher Nadeln.

Ausbeute: 2,91 g = 61 % der Theorie
Fp. : 191-193°C

Analog zu Beispiel 1.01 wurden die folgenden erfindungsgemäßen Verbindungen hergestellt:

| Beispiel | Name der Verbindung | Physikalische Konstante |
|---|---|---|
| 1.02 | 5-Hydroxy-1,3-dimethyl-4-(4-methoxymethyl-1,2,3-thiadiazol-yl-carbonyl)-pyrazol | Fp.: 168°C |
| 1.03 | 4-(4-Ethyl-1,2,3-thiadiazol-5-yl-carbonyl)-5-hydroxy-1,3-dimethyl-pyrazol | Fp.: 153°C |
| 1.04 | 5-Hydroxy-1-methyl-4-(4-methyl-1,2,3-thiadiazol-5-ylcarbonyl)-pyrazol | Fp.: 137-139°C |
| 1.05 | Gemisch aus 5-Hydroxy-1,3-dimethyl-4-(1,3-dimethyl-pyrazol-4-ylcarbonyl)-pyrazol und 5-Hydroxy-1,3-dimethyl-4-(1,5-dimethyl-pyrazol-4-ylcarbonyl)-pyrazol | Fp.: 191-192°C |
| 1.06 | 5-Hydroxy-4-(5-trifluormethyl-1-methyl-pyrazol-4-ylcarbonyl)-1,3-dimethylpyrazol | Fp.: 140-142°C |
| 1.07 | 5-Hydroxy-1,3-dimethyl-4-(4-methyl-thiazol-5-ylcarbonyl)-pyrazol | Fp.: 174-176°C |
| 1.08 | 5-Hydroxy-1,3-dimethyl-4-(2,4-dimethylthiazol-5-ylcarbonyl)-pyrazol | Fp.: 154-155°C |
| 1.09 | 5-Hydroxy-1,3-dimethyl-4-(4-methyl-2-phenyl-thiazol-5-ylcarbonyl)-pyrazol | Fp.: 175-176°C |

| Beispiel | Name der Verbindung | Physikalische Konstante |
|---|---|---|
| 1.10 | 5-Hydroxy-1-methyl-4-(2,4-dimethyl-thiazol-5-ylcarbonyl)-pyrazol | Fp.: 136-138°C |
| 1.11 | 4-(2-Chlor-4-methylthiazol-5-ylcarbonyl)-5-hydroxy-1,3-dimethylpyrazol | Fp.: 174°C |
| 1.12 | 4-(2-Chlor-5-methylthiazol-4-ylcarbonyl)-5-hydroxy-1,3-dimethylpyrazol | Fp.: 155°C |

**Beispiel 2.01**

5-Benzoyloxy-1,3-dimethyl-4-(4-methyl-1,2,3-thiadiazol-5-ylcarbonyl)-pyrazol

2,38 g (10 mmol) 5-Hydroxy-1,3-dimethyl-4-(4-methyl-1,2,3-thiadiazol-5-ylcarbonyl)-pyrazol, 1,59 g Natriumcarbonat und 1,4 ml Benzoylchlorid wurden in 60 ml tert.-Butanol 1,5 Stunden am Rückfluß erhitzt. Nach Abdestillieren des Lösungsmittels wurde in Dichlormethan aufgenommen, abgesaugt und das Filtrat eingeengt. Aus dem Rohprodukt wurde durch Säulenchromatographie an Kieselgel (Essigester/Hexan) die Titelverbindung in Form gelber Nadeln erhalten.
Ausbeute: 2,90 g = 84 % der Theorie
Fp.: 128°C

Analog zu Beispiel 2.01 wurden die folgenden erfindungsgemäßen Verbindungen hergestellt:

| Beispiel | Name der Verbindung | Physikalische Konstante |
|---|---|---|
| 2.02 | 5-(2,4-Dichlorbenzoyloxy)-1,3-dimethyl-4-(4-methyl-1,2,3-thiadiazol-5-ylcarbonyl)-pyrazol | Fp.: 152°C |
| 2.03 | 1,3-Dimethyl-5-(4-methylphenylsulfonyloxy)-4-(4-methyl-1,2,3-thiadiazol-5-ylcarbonyl)-pyrazol | Fp.: 137°C |
| 2.04 | 1,3-Dimethyl-5-(4-methylphenylsulfonyloxy)-4-(2,4-dimethyl-thiazol-5-ylcarbonyl)-pyrazol | Fp.: 103-105°C |
| 2.05 | 1,3-Dimethyl-5-methylsulfonyloxy-4-(2,4-dimethyl-thiazol-5-ylcarbonyl)-pyrazol | Fp.: 128-130°C |
| 2.06 | 1,3-Dimethyl-4-(2,4-dimethyl-thiazol-5-ylcarbonyl)-5-phenylsulfonyloxy-pyrazol | Fp.: 104-106°C |
| 2.07 | 5-Benzoyloxy-1,3-dimethyl-4-(2,4-dimethyl-thiazol-5-ylcarbonyl)-pyrazol | $n_D^{20}$: 1,5671 |
| 2.08 | 5-Benzoylmethoxy-1,3-dimethyl-4-(2,4-dimethyl-thiazol-5-ylcarbonyl)-pyrazol | $n_D^{20}$: 1,5972 |
| 2.09 | 4-(2,4-Dimethyl-thiazol-5-ylcarbonyl)-1-methyl-5-(4-methylphenylsulfonyloxy)-pyrazol | Fp.: 112-114 °C |

| Beispiel | Name der Verbindung | Physikalische Konstante |
|---|---|---|
| 2.10 | 5-(4-Chlorphenylsulfonyloxy)-1,3-dimethyl-4-(2,4-dimethylthiazol-5-ylcarbonyl)-pyrazol | Fp.: 106°C |
| 2.11 | 4-(2,4-Dimethylthiazol-5-ylcarbonyl)-5-(2,4,6-trimethylphenylsulfonyloxy)-1,3-dimethylpyrazol | Fp.: 124°C |
| 2.12 | 5-(4-Fluorphenylsulfonyloxy)-1,3-dimethyl-4-(2,4-dimethylthiazol-5-ylcarbonyl)-pyrazol | Fp.: 76°C |
| 2.13 | 5-(2,4,5-Trichlorphenylsulfonyloxy)-1,3-dimethyl-4-(2,4-dimethylthiazol-5-ylcarbonyl)-pyrazol | Fp.: 128°C |
| 2.14 | 5-(4-Methoxyphenylsulfonyloxy)-1,3-dimethyl-4-(2,4-dimethylthiazol-5-ylcarbonyl)-pyrazol | $n_D^{28}$: 1,5875 |
| 2.15 | 1,3-Dimethyl-4-(2,4-dimethylthiazol-5-ylcarbonyl)-5-(4-nitrophenylsulfonyloxy)-pyrazol | Fp.: 130°C |
| 2.16 | 1,3-Dimethyl-4-(2,4-dimethylthiazol-5-ylcarbonyl)-5-(4-nitrobenzoyloxy)-pyrazol | Fp.: 102°C |
| 2.17 | 1,3-Dimethyl-5-(4-methylbenzoyloxy)-4-(2,4-dimethylthiazol-5-ylcarbonyl)-pyrazol | Fp.: 80°C |
| 2.18 | (4-Chlorbenzoyloxy)-1,3-dimethyl-4-(2,4-dimethylthiazol-5-ylcarbonyl)-pyrazol | Fp.: 97-98°C |

| Beispiel | Name der Verbindung | Physikalische Konstante |
|---|---|---|
| 2.19 | 1,3-Dimethyl-(5-(4-methoxybenzoyl-oxy)-4-(2,4-dimethylthiazol-5-yl-carbonyl)-pyrazol | Fp.: 99-101°C |
| 2.20 | 5-(4-Fluorbenzoylmethoxy)-1,3-di-methyl-4-(2,4-dimethylthiazol-5-ylcarbonyl)-pyrazol | Fp.: 83-85°C |
| 2.21 | 5-(4-Brombenzoylmethoxy)-1,3-di-methyl-4-(2,4-dimethylthiazol-5-ylcarbonyl)-pyrazol | Fp.: 93-94°C |
| 2.22 | 5-(2,4-Dichlorbenzoylmethoxy)-1,3-dimethyl-4-(2,4-dimethylthiazol-5-ylcarbonyl)-pyrazol | Fp.: 70°C |
| 2.23 | 1,3-Dimethyl-4-(2,4-dimethylthi-azol-5-ylcarbonyl)-5-(2-naphthoyl-methoxy)-pyrazol | Fp.: 96-98°C |
| 2.24 | 5-(4-Chlorbenzoylmethoxy)-1,3-di-methyl-4-(2,4-dimethylthiazol-5-ylcarbonyl)-pyrazol | Fp.: 95-97°C |
| 2.25 | 5-(4-Methylbenzoylmethoxy)-1,3-dimethyl-4-(2,4-dimethylthiazol-5-ylcarbonyl)-pyrazol | Fp.: 88-90°C |
| 2.26 | 4-(2-Chlor-4-methylthiazol-5-yl-carbonyl)-1,3-dimethyl-5-phenyl-sulfonyloxypyrazol | Fp.: 138°C |
| 2.27 | 4-(2-Chlor-4-methylthiazol-5-yl-carbonyl)-1,3-dimethyl-5-(4-methyl-phenylsulfonyloxy)-pyrazol | Fp.: 94-96°C |

| Beispiel | Name der Verbindung | Physikalische Konstante |
|---|---|---|
| 2.28 | 4-(2-Chlor-4-methylthiazol-5-yl-carbonyl)-5-benzoylmethoxy-1,3-dimethylpyrazol | $n_D^{21}$: 1,5967 |
| 2.29 | 4-(2-Chlor-5-methylthiazol-4-yl-carbonyl)-1,3-dimethyl-5-(4-methyl-sulfonyloxy)-pyrazol | Fp.: 104-106°C |
| 2.30 | 1-Methyl-5-(4-methylphenylsulfonyl-oxy)-4-(2,4-dimethylthiazol-5-ylcarbonyl)-pyrazol | Fp.: 114°C |

**Beispiel 3.01**

5-Chlor-1,3-dimethyl-4-(4-methyl-1,2,3-thiadiazol-5-ylcarbonyl)-pyrazol

Zu 3,89 g (16,3 mmol) 5-Hydroxy-1,3-dimethyl-4-(4-methyl-1,2,3-thiadiazol-5-ylcarbonyl)-pyrazol und 21 ml Oxalylchlorid in 30 ml Dichlormethan wurde tropfenweise soviel Dimethylformamid gegeben, bis kein Aufschäumen mehr zu beobachten war. Nach 4 Stunden Rühren bei 25°C wurde in Wasser/Dichlormethan aufgenommen, die organische Phase abgetrennt, getrocknet (Magnesiumsulfat) und einrotiert. Umkristallisation des Rückstandes aus Hexan lieferte gelbe Kristalle.
Ausbeute: 3,30 g = 70 % der Theorie
Fp.: 105-108°C

**Beispiel 4.01**

L-2-[1,3-Dimethyl-4-(4-methyl-1,2,3-thiadiazol-5-ylcarbonyl)-pyrazol-5-yl-amino]-propansäuremethylester

Zu 3,85 g (15 mmol) 5-Chlor-1,3-dimethyl-4-(4-methyl-1,2,3-thiadiazol-5-ylcarbonyl)-pyrazol und 10 ml Triethylamin in 80 ml Dichlormethan wurden bei 0°C 2,09 g (15 mmol) L-Alaninmethylester-Hydrochlorid gegeben und anschließend noch 3 Stunden bei Raumtemperatur gerührt. Das Reaktionsgemisch wurde mit Wasser gewaschen, getrocknet (Magnesiumsulfat) und einrotiert. Filtration über Kieselgel (Essigester/Hexan) liefert der Titelverbindung in kristalliner Form.
Ausbeute: 1,80 g = 37 % der Theorie
Fp.: 122°C

Analog zu Beispiel 4.01 wurden die folgenden erfindungsgemäßen Verbindungen hergestellt:

| Beispiel Nr. | Name der Verbindung | Physikalische Konstante |
|---|---|---|
| 4.02 | 1,3-Dimethyl-5-methylamino-4-(4-methyl-1,2,3-thiadiazol-5-yl-carbonyl)-pyrazol | Fp.: 178°C |
| 4.03 | 5-Allylamino-1,3-dimethyl-4-(4-methyl-1,2,3-thiadiazol-5-ylcarbonyl)-pyrazol | Fp.: 142°C |
| 4.04 | 5-Isopropylamino-1,3-dimethyl-4-(4-methyl-1,2,3-thiadiazol-5-yl-carbonyl)-pyrazol | Fp.: 131°C |
| 4.05 | 1,3-Dimethyl-4-(4-methyl-1,2,3-thiadiazol-5-ylcarbonyl)-5-propylaminopyrazol | Fp.: 133°C |
| 4.06 | 1,3-Dimethyl-5-(2-dimethylaminoethylamino-4-(4-methyl-1,2,3-thiadiazol-5-ylcarbonyl)-pyrazol | Fp.: 103°C |
| 4.07 | 5-Ethylmercapto-1,3-dimethyl-4-(4-methyl-1,2,3-thiadiazol-5-ylcarbonyl)-pyrazol | Fp.: 142-143°C |
| 4.08 | 5-Allylamino-1,3-dimethyl-4-(2,4-dimethylthiazol-5-ylcarbonyl)-pyrazol | Fp.: 93°C |
| 4.09 | 1,3-Dimethyl-4-(2,4-dimethylthiazol-5-ylcarbonyl)-5-phenylmercaptopyrazol | Fp.: 114°C |

Die folgenden Beispiele erläutern die Anwendungsmöglichkeiten der erfindungsgemäßen Verbindungen.

**Beispiel A**

Im Gewächshaus wurden die aufgeführten Pflanzenspezies nach dem Auflaufen mit den aufgeführten Verbindungen in einer Aufwandmenge von 0,3 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigte zwei Wochen nach der Behandlung die erfindungsgemäße Verbindung eine hohe Kulturpflanzenselektivität in Brassica sp. bei ausgezeichneter Wirkung gegen das Unkraut. Das Vergleichsmittel zeigte nicht die gleichhohe Wirksamkeit.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
BRSSS = Brassica sp.
ALOMY = Alopecurus myosuroides
SETVI = Setaria viridis
SORHA = Sorghum halepense
ABUTH = Abutilon theophrasti
GALAP = Galium aparine
POLSS = Polygonum sp.
VIOSS = Viola sp.

| Erfindungsgemäße Verbindung | BRSSS | ALOMY | SETVI | SORHA | ABUTH | GALAP | POLSS | VIOSS |
|---|---|---|---|---|---|---|---|---|
| Beispiel 1.08 | 1 | 3 | 3 | 3 | 3 | 3 | 3 | 3 |
| Unbehandelt | 0 | 0 | 0 | 0 | 0 | 0 | 0 | 0 |
| Vergleichsmittel | | | | | | | | |
| 5-Hydroxy-1,3-dimethyl-4-(2,4-dichlor-benzoyl)-pyrazol | 0 | 1 | 2 | 2 | 0 | 0 | 0 | 1 |

**Beispiel B**

Im Gewächshaus wurden die in der Tabelle aufgeführten Verbindungen mit den ebenfalls erwähnten

Aufwandmengen appliziert. Hierzu wurden die Wirkstoffe in Form von Zubereitungen auf die Wasseroberfläche in Gefäße mit 1500 ml Wasser pipettiert. Die Testpflanzenarten wurden im 2- bis 5-Blatt-Stadium eingesetzt. Drei Wochen nach der Applikation wurde die Schädigung der Pflanzen bonitiert. Die erfindungsgemäße Verbindung zeigte eine starke Wirkung gegen wichtige Reisunkräuter bei gleichzeitiger Selektivität in Wasserreis. Das Vergleichsmittel war schwächer wirksam.

In der folgenden Tabelle bedeuten:
0 = keine Schädigung
1 = schwache Schädigung
2 = mittlere Schädigung
3 = starke Schädigung
4 = total vernichtet
ORYSA = Oryza sativa
ECHCG = Echinochloa crus-galli
SAGPY = Sagittaria pygmaea
SCPJU = Scirpus juncoides
MOOVA = Monochoria vaginalis
CYPSE = Cyperus serotinus

| Erfindungsgemäße Verbindung | Wasserapplikation kg Wirkstoff/ha | ORYSA | ECHCG | SAGPY | SCPJU | MOOVA | CYPSE |
|---|---|---|---|---|---|---|---|
| Beispiel 1.08 | 1,0 | 0 | 4 | 4 | 4 | 4 | 4 |
| Vergleichsmittel | | | | | | | |
| 1,3-Dimethyl-5-(4-methyl-phenylsulfonyloxy)-4-(2,4-dichlorbenzoyl)-pyrazol | 1,0 | 0 | 3 | 4 | 3 | 3 | 3 |

**Beispiel C**

Im Gewächshaus wurden die in der Tabelle aufgeführten Verbindungen mit den ebenfalls erwähnten Aufwandmengen appliziert. Hierzu wurden die Wirkstoffe in Form von Zubereitungen auf die Wasseroberfläche in Gefäße mit 1500 ml Wasser pipettiert. Die Testpflanzenarten wurden im Vorauflauf und im 1- bis 5-Blatt-Stadium eingesetzt. Drei Wochen nach der Applikation wurde die Schädigung der Pflanzen bonitiert. Die erfindungsgemäßen Verbindungen zeigten eine starke Wirkung gegen wichtige Reisunkräuter bei gleichzeitiger Selektivität in Wasserreis. Das Vergleichsmittel zeigte nicht die gleichhohe Wirksamkeit.

In der folgenden Tabelle bedeuten:
0 = keine Schädigung
1 = schwache Schädigung
2 = mittlere Schädigung
3 = starke Schädigung
4 = total vernichtet
ORYSA = Oryza sativa
ECHCG = Echinochloa crus-galli
SAGPY = Sagittaria pygmaea

SCPJU = Scirpus juncoides
MOOVA = Monochoria vaginalis
CYPSE = Cyperus serotinus
PASDS = Paspalum distichum

| Erfindungsgemäße Verbindung | Wasserapplikation kg Wirkstoff/ha | ORYSA | ECHCG | SAGPY | SCPJU | MOOVA | CYPSE | PASDS |
|---|---|---|---|---|---|---|---|---|
| Beispiel 1.04 | 0,5 | 0 | - | - | 3 | 4 | - | 4 |
| Beispiel 1.07 | 0,5 | 0 | - | 2 | - | - | 2 | 2 |
| Beispiel 1.08 | 0,5 | 0 | 3 | 4 | 4 | 4 | - | 4 |
| Beispiel 1.10 | 0,5 | 0 | 2 | - | - | 4 | - | 2 |
| Beispiel 1.11 | 0,5 | 0 | - | - | 3 | - | - | - |
| Beispiel 2.04 | 0,5 | 0 | 3 | 4 | - | 3 | 4 | 3 |
| Beispiel 2.05 | 0,5 | 0 | - | 3 | - | 3 | 4 | 3 |
| Beispiel 2.06 | 0,5 | 0 | 3 | - | 3 | 3 | 3 | 3 |
| Beispiel 2.07 | 0,5 | 0 | 4 | 4 | 4 | 4 | - | 4 |
| Beispiel 2.08 | 0,5 | 0 | 3 | 4 | - | 4 | 4 | 3 |
| Beispiel 2.10 | 0,5 | 0 | 3 | - | 2 | 3 | 2 | 2 |
| Beispiel 2.11 | 0,5 | 0 | 3 | - | 3 | 3 | 3 | 2 |
| Beispiel 2.12 | 0,5 | 0 | 3 | - | 3 | 3 | 2 | 2 |
| Beispiel 2.13 | 0,5 | 0 | 3 | - | 2 | 2 | 2 | - |
| Beispiel 2.14 | 0,5 | 0 | 3 | - | 3 | 3 | 3 | 2 |
| Beispiel 2.15 | 0,5 | 0 | 3 | - | 3 | 3 | 2 | 2 |
| Beispiel 2.16 | 0,5 | 0 | 2 | - | 2 | 3 | 3 | 2 |
| Beispiel 2.17 | 0,5 | 0 | 3 | - | 3 | 3 | 3 | 3 |
| Beispiel 2.18 | 0,5 | 0 | 2 | - | 3 | 3 | 2 | 3 |
| Beispiel 2.19 | 0,5 | 0 | 3 | - | 3 | 3 | 3 | 2 |
| Beispiel 2.20 | 0,5 | 0 | 3 | - | 4 | 3 | 2 | 2 |
| Beispiel 2.21 | 0,5 | 0 | 3 | - | 3 | 2 | 2 | 2 |
| Beispiel 2.24 | 0,5 | 0 | 3 | - | 3 | 3 | 3 | 2 |
| Beispiel 2.25 | 0,5 | 0 | 3 | - | 3 | 3 | 3 | 2 |
| Beispiel 2.27 | 0,5 | 0 | 2 | - | 3 | 3 | - | 2 |
| Beispiel 2.28 | 0,5 | 0 | 2 | - | 3 | 3 | - | - |
| Beispiel 4.09 | 0,5 | 0 | 2 | - | 3 | 3 | 2 | 2 |
| Unbehandelt | | 0 | 0 | 0 | 0 | 0 | 0 | 0 |
| **Vergleichsmittel** | | | | | | | | |
| Pyrazolate | 0,5 | 0 | 1 | - | 2 | - | 1 | 2 |

**Beispiel D**

Im Gewächshaus wurden die aufgeführten Pflanzenspezies vor dem Auflaufen mit den aufgeführten Verbindungen in einer Aufwandmenge von 3,0 kg Wirkstoff/ha behandelt. Die Verbindungen wurden zu diesem Zweck als Emulsionen oder Suspensionen mit 500 Litern Wasser/ha gleichmäßig über den Boden versprüht. Hier zeigten drei Wochen nach der Behandlung die erfindungsgemäßen Verbindungen eine hohe Kulturpflanzenselektivität in Soja und Weizen bei ausgezeichneter Wirkung gegen das Unkraut.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
GLXMA = Glycine maxima
TRZAX = Triticum aestivum
BEAVX = Beta vulgaris altissima
HELAN = Helianthus annuus
ALOMY = Alopecurus myosuroides
AVEFA = Avena fatua
SETVI = Setaria viridis
PANSS = Panicum maximum
CYPES = Cyperus esculentus
ABUTH = Abutilon theophrasti
GALAP = Galium aparine
MATCH = Matricaria chamomilla
POLSS = Polygonum sp.
SEBEX = Sesbania exaltata
SOLSS = Solanum sp.
VERPE = Veronica persica
VIOSS = Viola sp.

| Erfindungsgemäße Verbindungen | GLXMA | TRZAX | BEAVX | HELAN | ALOMY | AVEFA | SETVI | PANSS | CYPES | ABUTH | GALAP | MATCH | POLSS | SEBEX | SOLSS | VERPE | VIOSS |
|---|---|---|---|---|---|---|---|---|---|---|---|---|---|---|---|---|---|
| Beispiel 1.01 | 0 | 0 | 3 | 3 | 3 | 3 | 3 | 4 | 4 | 3 | - | - | 3 | 3 | - | 3 | - |
| Beispiel 1.03 | - | 0 | 4 | 3 | 3 | - | - | 3 | 4 | - | - | 4 | - | - | 3 | - | - |
| Beispiel 1.04 | 0 | 0 | 4 | - | - | - | 4 | - | 3 | - | - | - | - | - | - | - | - |
| Beispiel 1.06 | - | 0 | 4 | - | - | - | 3 | - | 3 | - | 3 | 3 | 3 | 3 | 4 | 4 | 4 |
| Unbehandelt | 0 | 0 | 0 | 0 | 0 | 0 | 0 | 0 | 0 | 0 | 0 | 0 | 0 | 0 | 0 | 0 | 0 |

**Beispiel E**

Im Gewächshaus wurden die aufgeführten Pflanzenspezies nach dem Auflaufen mit den aufgeführten

Verbindungen in einer Aufwandmenge von 3,0 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigten zwei Wochen nach der Behandlung die erfindungsgemäßen Verbindungen eine hohe Kulturpflanzenselektivität in Mais und Weizen bei ausgezeichneter Wirkung gegen das Unkraut.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
ZEAMX = Zea mays
TRZAX = Triticum aestivum
BEAVX = Beta vulgaris altissima
GLXMA = Glycine maxima
ALOMY = Alopecurus myosuroides
SETVI = Setaria viridis
PANSS = Panicum maximum
SORHA = Sorghum halepense
CYPES = Cyperus esculentus
ABUTH = Abutilon theophrasti
GALAP = Galium aparine
IPOSS = Ipomoea purpurea
MATCH = Matricaria chamomilla
POLSS = Polygonum sp.
VERPE = Veronica persica
VIOSS = Viola sp.

| Erfindungsgemäße Verbindungen | ZEAMX | TRZAX | BEAVX | GLXMA | ALOMY | SETVI | PANSS | SORHA | CYPES | ABUTH | GALAP | IPOSS | MATCH | POLSS | SEBEX | SOLSS | VERPE | VIOSS |
|---|---|---|---|---|---|---|---|---|---|---|---|---|---|---|---|---|---|---|
| Beispiel 2.05 | 0 | 0 | 3 | 3 | - | 3 | 3 | 3 | 3 | 3 | 3 | - | 3 | 3 | 3 | 3 | 3 | 3 |
| Beispiel 2.04 | - | 0 | 4 | 3 | 3 | 3 | - | - | - | 3 | 3 | 3 | 3 | 3 | 3 | 3 | 3 | 3 |
| Unbehandelt | 0 | 0 | 0 | 0 | 0 | 0 | 0 | 0 | 0 | 0 | 0 | 0 | 0 | 0 | 0 | 0 | 0 | 0 |

## Ansprüche

1. Substituierte 4-Heteroaroylpyrazole der allgemeinen Formel I

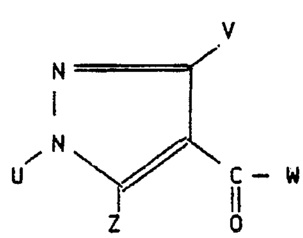

(I) ,

in der
U ein Wasserstoffatom, einen $C_1$-$C_4$-Alkylrest oder einen Halogen-$C_1$-$C_4$-alkylrest,
V ein Wasserstoffatom, einen $C_1$-$C_4$-Alkylrest oder einen Halogen-$C_1$-$C_4$-alkylrest,
W eine heterocyclische Gruppe W-1 bis W-7 der allgemeinen Formeln

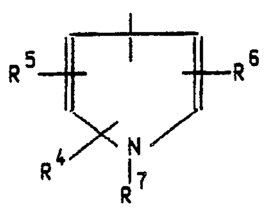 , 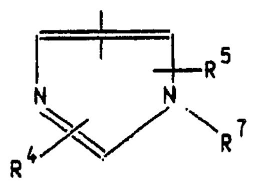 , 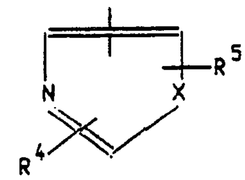 ,

W - 1 W - 2 W - 3

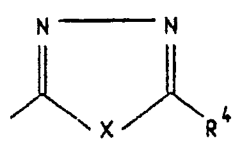 , 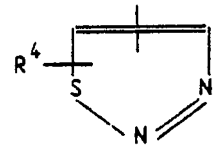 , 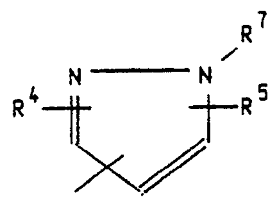

W - 4 W - 5 W - 6

oder 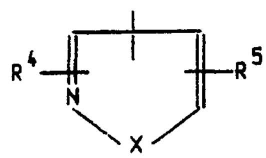

W - 7

X ein Sauerstoffatom oder ein Schwefelatom,
Z ein Halogenatom oder eine der Gruppen $-OR^1$, $-O-(CH_2)_n-CO-R^1$, $-O-SO_2-R^1$. $-S(O)_n-R^1$, -OM oder $-NR^2R^3$,
n 0, 1 oder 2,
M ein Kation aus der Gruppe Lithium, Natrium und Kalium, ein Äquivalent aus der Gruppe Zink, Mangan, Calcium, Magnesium und Barium oder ein Ammoniumion der allgemeinen Formel

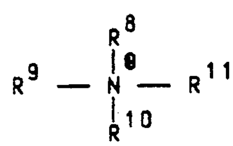 ,

$R^1$ ein Wasserstoffatom, einen $C_1$-$C_{18}$-Alkylrest, einen ein- oder mehrfach, gleich oder verschieden durch Halogen, Hydroxy oder Cyano substituierten oder durch Sauerstoff oder Schwefel ein- oder mehrfach unterbrochenen $C_1$-$C_{18}$-Alkylrest, einen $C_2$-$C_{12}$-Alkenylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkinylrest, einen Phenylrest, einen ein- oder mehrfach, gleich oder verschieden durch Halogen, Nitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenylrest, einen Phenyl-$C_1$-$C_3$-alkylrest oder einen ein-oder mehrfach, gleich oder verschieden durch Halogen, Nitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenyl-$C_1$-$C_3$-alkylrest,
$R^2$ ein Wasserstoffatom, einen $C_1$-$C_{18}$-Alkylrest, einen ein- oder mehrfach, gleich oder verschieden durch Halogen, Hydroxy, Cyano, $C_1$-$C_4$-Alkoxycarbonyl, einen Rest $-NR^{12}R^{13}$ oder einen Rest $-N^{\oplus}R^{12}R^{13}R^{14}$

substituierten oder durch Sauerstoff oder Schwefel ein- oder mehrfach unterbrochenen $C_1$-$C_{18}$-Alkylrest, einen $C_2$-$C_{12}$-Alkenylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkinylrest, einen $C_1$-$C_6$-Alkoxyrest, einen Halogen-$C_1$-$C_6$-alkoxy rest, einen Phenylrest, einen ein- oder mehrfach, gleich oder verschieden durch Halogen, Nitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenylrest, einen Phenyl-$C_1$-$C_3$-alkylrest oder einen ein- oder mehrfach, gleich oder verschieden durch Halogen, Nitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenyl-$C_1$-$C_3$-alkylrest,

$R^3$ ein Wasserstoffatom, einen $C_1$-$C_{18}$-Alkylrest, einen ein- oder mehrfach, gleich oder verschieden durch Halogen, Hydroxy, Cyano, $C_1$-$C_4$-Alkoxycarbonyl, einen Rest $-NR^{12}R^{13}$ oder einen Rest $-N^{\oplus}R^{12}R^{13}R^{14}$ substituierten oder durch Sauerstoff oder Schwefel ein- oder mehrfach unterbrochenen $C_1$-$C_{18}$-Alkylrest, einen $C_2$-$C_{12}$-Alkenylrest, einen ein-oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein-oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkinylrest, einen Phenylrest, einen ein- oder mehrfach, gleich oder verschieden durch Halogen, Nitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenyl-rest, einen Phenyl-$C_1$-C3-alkylrest oder einen ein- oder mehrfach, gleich oder verschieden durch Halogen, Nitro, Cyano, $C_1$-$C_4$-Alkyl, Halogen-$C_1 C_4$-alkyl, $C_1$-$C_4$-Alkoxy oder Halogen-$C_1$-$C_4$-alkoxy substituierten Phenyl-$C_1$-$C_3$-alkylrest oder

$R^2$ und $R^3$ gemeinsam mit dem benachbarten Stickstoffatom eine Morpholinogruppe, eine Piperidinogruppe oder eine Pyrrolidinogruppe,

$R^4$ ein Wasserstoffatom, ein Halogenatom, einen $C_1$-$C_{12}$-Alkylrest, einen durch Sauerstoff oder Schwefel ein- oder mehrfach unterbrochenen $C_2$-$C_{12}$-Alkylrest, einen Halogen-$C_1$-$C_4$-alkylrest, einen $C_1$-$C_4$-Alkoxyrest, einen Halogen-$C_1$-$C_4$-alkoxyrest, einen $C_1$-$C_4$-Alkylmercaptorest, einen $C_1$-$C_4$-Alkoxycarbonylrest, einen Phenylrest, einen durch Halogen, Nitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenylrest, eine Cyanogruppe oder eine Nitrogruppe,

$R^5$ ein Wasserstoffatom, ein Halogenatom, einen $C_1$-$C_{12}$-Alkylrest, einen durch Sauerstoff oder Schwefel ein- oder mehrfach unterbrochenen $C_2$-$C_{12}$-Alkylrest, einen Halogen-$C_1$-$C_4$-alkylrest, einen $C_1$-$C_4$-Alkoxyrest, einen Halogen-$C_1$-$C_4$-alkoxyrest, einen $C_1$-$C_4$-Alkylmercaptorest, einen $C_1$-$C_4$-Alkoxycarbonylrest, einen Phenylrest, einen durch Halogen, Nitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenylrest, eine Cyanogruppe oder eine Nitrogruppe,

$R^6$ ein Wasserstoffatom, ein Halogenatom, einen $C_1$-$C_{12}$-Alkylrest, einen durch Sauerstoff oder Schwefel ein- oder mehrfach unterbrochenen $C_2$-$C_{12}$ Alkylrest, einen Halogen-$C_1$-$C_4$-alkylrest, einen $C_1$-$C_4$-Alkoxyrest, einen Halogen-$C_1$-$C_4$-alkoxyrest, einen $C_1$-$C_4$-Alkylmercaptorest, einen $C_1$-$C_4$-Alkoxycarbonylrest, einen Phenylrest, einen durch Halogen, Nitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenylrest, eine Cyanogruppe oder eine Nitrogruppe,

$R^7$ ein Wasserstoffatom, ein Halogenatom, einen $C_1$-$C_{12}$-Alkylrest, einen durch Sauerstoff oder Schwefel ein- oder mehrfach unterbrochenen $C_2$-$C_{12}$-Alkylrest, einen Halogen-$C_1$-$C_4$-alkylrest, einen $C_1$-$C_4$-Alkoxyrest, einen Halogen-$C_1$-$C_4$-alkoxyrest, einen $C_1$-$C_4$-Alkylmercaptorest, einen $C_1$-$C_4$-Alkoxycarbonylrest, einen Phenylrest, einen durch Halogen, Nitro, Cyano, $C_1$-$C_4$-Alkyl, Halogen-$C_1$-$C_4$-alkyl, $C_1$-$C_4$-Alkoxy oder Halogen-$C_1$-$C_4$-alkoxy substituierten Phenylrest, eine Cyanogruppe oder eine Nitrogruppe,

$R^8$ ein Wasserstoffatom, einen $C_1$-$C_{12}$-Alkylrest, einen durch Sauerstoff oder Schwefel ein- oder mehrfach unterbrochenen $C_2$-$C_{12}$-Alkylrest, einen Phenylrest oder einen Benzylrest,

$R^9$ ein Wasserstoffatom, einen $C_1$-$C_{12}$-Alkylrest, einen durch Sauerstoff oder Schwefel ein- oder mehrfach unterbrochenen $C_2$-$C_{12}$-Alkylrest, einen Phenylrest oder einen Benzylrest,

$R^{10}$ ein Wasserstoffatom, einen $C_1$-$C_{12}$-Alkylrest, einen durch Sauerstoff oder Schwefel ein- oder mehrfach unterbrochenen $C_2$-$C_{12}$-Alkylrest, einen Phenylrest oder einen Benzylrest,

$R^{11}$ ein Wasserstoffatom, einen $C_1$-$C_{12}$-Alkylrest, einen durch Sauerstoff oder Schwefel ein- oder mehrfach unterbrochenen $C_2$-$C_{12}$-Alkylrest, einen Phenylrest oder einen Benzylrest,

$R^{12}$ ein Wasserstoffatom oder einen $C_1$-$C_{12}$-Alkylrest,

$R^{13}$ ein Wasserstoffatom oder einen $C_1$-$C_{12}$-Alkylrest und

$R^{14}$ ein Wasserstoffatom oder einen $C_1$-$C_{12}$-Alkylrest

bedeuten.

2. Verfahren zur Herstellung von Verbindungen der allgemeinen Formel I gemäß Anspruch 1, dadurch gekennzeichnet, daß man

A) falls Z eine Hydroxygruppe bedeutet, eine Verbindung der allgemeinen Formel II

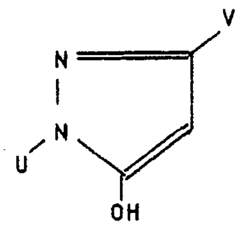

(II) ,

in der U und V die unter der allgemeinen Formel I genannten Bedeutungen haben, mit einem Carbonsäurederivat der allgemeinen Formel III

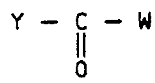

(III) ,

in der W die unter der allgemeinen Formel I genannten Bedeutungen hat und Y für ein Halogenatom, vorzugsweise ein Chloratom oder ein Bromatom, oder einen $C_1$-$C_4$-Alkoxyrest steht, in Anwesenheit einer geeigneten Base oder einer Lewis-Säure zur Reaktion bringt oder

B) falls Z eine Hydroxygruppe bedeutet, eine Verbindung der allgemeinen Formel II

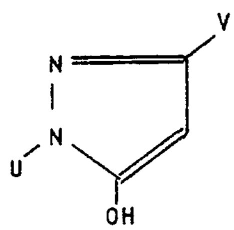

(II) ,

in der U und V die unter der allgemeinen Formel I genannten Bedeutungen haben, mit einer Carbonsäure der allgemeinen Formel IV

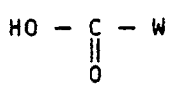

(IV) ,

in der W die unter der allgemeinen Formel I genannten Bedeutungen hat, in Anwesenheit eines wasserentziehenden Mittels und einer geeigneten Base oder einer Lewis-Säure zur Reaktion bringt oder

C) falls Z für die Gruppe $-OR^1$ steht, eine Verbindung der allgemeinen Formel V

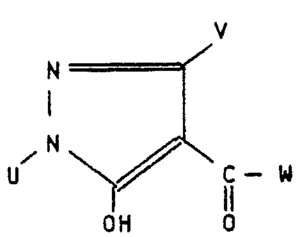

(V) ,

in der U, V und W die unter der allgemeinen Formel I genannten Bedeutungen haben, mit einem Alkylierungsmittel der allgemeinen Formel VI

$R^1$ - Y (VI) ,

in der $R^1$ die unter der allgemeinen Formel I genannten Bedeutungen hat und Y für die unter der allgemeinen Formel III angegebenen Bedeutungen steht, umsetzt oder
D) falls Z für die Gruppe $-O-(CH_2)_n-CO-R^1$ steht, eine Verbindung der allgemeinen Formel V

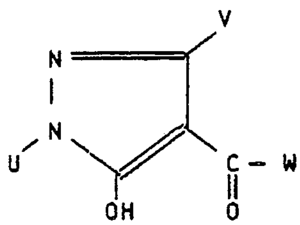

(V) ,

in der U, V und W die unter der allgemeinen Formel I genannten Bedeutungen haben, mit einem Alkylierungsmittel oder einem Acylierungsmittel der allgemeinen Formel VII
$R^1-CO-(CH_2)_n-Y$ (VIII) ,
in der $R^1$ und n die unter der allgemeinen Formel I genannten Bedeutungen haben und Y für die unter der allgemeinen Formel III angegebenen Bedeutungen steht, umsetzt oder
E) falls Z für die Gruppe $-O-SO_2-R^1$ steht, eine Verbindung der allgemeinen Formel V

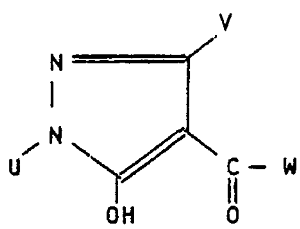

(V) ,

in der U, V und W die unter der allgemeinen Formel I genannten Bedeutungen haben, mit einem Sulfonylierungsmittel der allgemeinen Formel VIII
$R^1-SO_2-Y$ (VIII) ,
in der $R^1$ die unter der allgemeinen Formel I genannten Bedeutungen hat und Y für die unter der allgemeinen Formel III angegebenen Bedeutungen steht, umsetzt oder
F) falls Z für die Gruppe -OM steht, eine Verbindung der allgemeinen Formel V

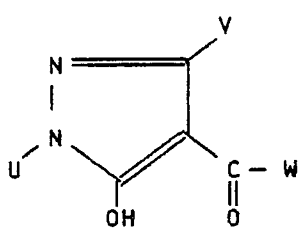

(V) ,

in der U, V und W die unter der allgemeinen Formel I genannten Bedeutungen haben, mit einer Base der allgemeinen Formel IX
M - Q (IX) ,
in der M die unter der allgemeinen Formel I angegebenen Bedeutungen hat und Q für eine Hydroxygruppe oder ein Carbonatäquivalent steht, umsetzt oder
G) falls Z für ein Halogenatom steht, eine Verbindung der allgemeinen Formel V

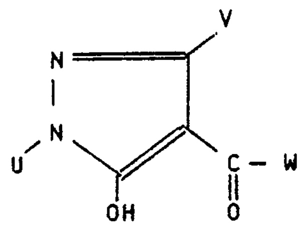

(V) ,

in der U, V und W die unter der allgemeinen Formel I genannten Bedeutungen haben, nach an sich bekannten Verfahren halogeniert oder

H) falls Z für die Gruppe $-NR^2R^3$ steht, eine Verbindung der allgemeinen Formel X

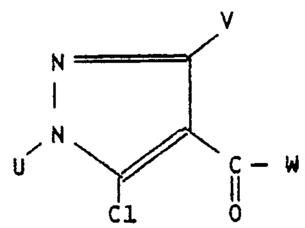

(X) ,

in der U, V und W die unter der allgemeinen Formel I genannten Bedeutungen haben, mit einem Amin der allgemeinen Formel XI

$HNR^2R^3$ (XI) ,

in der $R^2$ und $R^3$ die unter der allgemeinen Formel I genannten Bedeutungen haben, umsetzt oder

I) falls Z für die Grupppe $-S(O)_n-R^1$ steht, eine Verbindung der allgemeinen Formel X

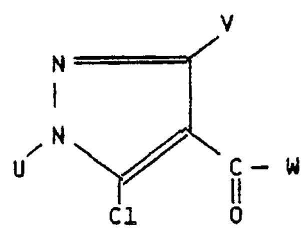

(X) ,

in der U, V und W die unter der allgemeinen Formel I genannten Bedeutungen haben, mit einem Thioalkohol der allgemeinen Formel XII

$HSR^1$ (XII) ,

in der $R^1$ die unter der allgemeinen Formel I genannten Bedeutungen hat, umsetzt und das Reaktionsprodukt gewünschtenfalls anschließend oxydiert.

3. Mittel mit herbizider Wirkung, gekennzeichnet durch einen Gehalt an mindestens einer Verbindung gemäß dem Anspruch 1.

4. Verwendung von Mitteln gemäß dem Anspruch 3 zur Bekämpfung monokotyler und dikotyler Unkrautarten in landwirtschaftlichen Hauptkulturen.

5. Verfahren zur Herstellung von Mitteln mit herbizider Wirkung, dadurch gekennzeichnet, daß man Verbindungen der allgemeinen Formel I gemäß dem Anspruch 1 mit Träger- und/oder Hilfsstoffen vermischt.

Europäisches Patentamt

**EUROPÄISCHER TEILRECHERCHENBERICHT,** der nach Regel 45 des Europäischen Patent-übereinkommens für das weitere Verfahren als europäischer Recherchenbericht gilt

Nummer der Anmeldung

EP 90 25 0189

**EINSCHLÄGIGE DOKUMENTE**

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 77, Nr. 13, 25. September 1972, Seite 452, Zusammenfassung Nr. 88384b, Columbus, Ohio, US; A.S. SARENKO et al.: "Heterocyclic analogs of xanthones. II. C-Acylation of 5-pyrazolinones and synthesis of chromono [3,2-d] pyrazoles", & KHIM. GETEROTSIKL. SOEDIN. 1972, (6), 799-804<br>* Zusammenfassung *<br>-- | 1 | C 07 D 417/06<br>A 01 N 43/56<br>C 07 D 413/06<br>C 07 D 403/06<br>C 07 D 231/20<br>A 01 N 43/78<br>A 01 N 43/82<br>A 01 N 43/76<br>A 01 N 43/50<br>A 01 N 43/80 |
| A | CHEMICAL ABSTRACTS, Band 93, Nr. 3, 21. Juli 1980, Seite 208, Zusammenfassung Nr. 20584s, Columbus, Ohio, US; L.V. GANGAWANE et al.: "In vitro stimulation and inhibition of groundnut rhizobium by 4-aroyl-3,5-diaryl pyrazoles", & SCI. CULT. 1979; 45(9), 369-70<br>./. | | |

| RECHERCHIERTE SACHGEBIETE (Int. Cl.4) |
|---|
| C 07 D 417/00<br>A 01 N 43/00<br>C 07 D 413/00<br>C 07 D 403/00<br>C 07 D 231/00 |

**UNVOLLSTÄNDIGE RECHERCHE**

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche:

Unvollständig recherchierte Patentansprüche:

Nicht recherchierte Patentansprüche:

Grund für die Beschränkung der Recherche:

Siehe Blatt -C-

| Recherchenort | Abschlußdatum der Recherche | Prüfer |
|---|---|---|
| DEN HAAG | 29-10-1990 | BUYSER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 06.82

Europäisches Patentamt

# EUROPÄISCHER TEILRECHERCHENBERICHT

Nummer der Anmeldung

EP 90 25 0189
-2-

| EINSCHLÄGIGE DOKUMENTE | | | KLASSIFIKATION DER ANMELDUNG (Int. Cl. 4) |
|---|---|---|---|
| Kategorie | Kennzeichnung des Dokuments mit Angabe, soweit erforderlich, der maßgeblichen Teile | betrifft Anspruch | |
| | * Zusammenfassung * <br> -- | | |
| A | CHEMICAL ABSTRACTS, Band 92, Nr. 21, 26. Mai 1980, Seite 165, Zusammenfassung Nr. 175648u, Columbus, Ohio, US; K. KAWAKUBO et al.: "A mechanism of chlorosis caused by 1,3-dimethyl-4-(2,4-dichlorobenzoyl)-5-hydroxypyrazole, a herbicidal compound", & PLANT PHYSIOL. 1979, 64(5), 774-9 <br> * Zusammenfassung * <br> -- | | RECHERCHIERTE SACHGEBIETE (Int. Cl. 4) |
| A | CHEMICAL ABSTRACTS, Band 92, Nr. 7, 18. Februar 1980, Seite 670, Zusammenfassung Nr. 58723d, Columbus, Ohio, US; F. EIDEN et al.: "Studies on pyrone derivatives. Part 80. Pyridone, pyrazole and pyrimidine derivatives from 3,5-diacyl-4-pyrones", & ARCH. PHARM. (WEINHEIM, GER.) 1979, 312(10), 863-72 <br> * Zusammenfassung * <br> -- | | |
| A | CHEMICAL ABSTRACTS, Band 88, Nr. 21, 22. Mai 1978, Seite 597, Zusammenfassung Nr. 152485s, Columbus, Ohio, US; K.A. THAKAR et al.: "Synthesis of 4-aroyl-3,5-diarylpyrazoles", & INDIAN J. CHEM., SECT. B 1977, 15B(11), 1059-61 <br> * Zusammenfassung * <br> -- <br> ./. | | |

Europäisches Patentamt

# EUROPÄISCHER TEILRECHERCHENBERICHT

Nummer der Anmeldung
EP 90 25 0189
-3-

| EINSCHLÄGIGE DOKUMENTE | | | KLASSIFIKATION DER ANMELDUNG (Int. Cl.4) |
|---|---|---|---|
| Kategorie | Kennzeichnung des Dokuments mit Angabe, soweit erforderlich, der maßgeblichen Teile | betrifft Anspruch | |
| A | JOURNAL OF HETEROCYCLIC CHEMISTRY, Band 21, Juli-August 1984, Seiten 1175-1176, Provo, US; H.A. DeWALD: "Synthesis of 5-amino-1,3-dimethylpyrazol-4-yl aryl ketones"<br>* Der ganze Artikel *<br>-- | | |
| A | JOURNAL OF MEDICINAL CHEMISTRY, Band 30, Nr. 10, Oktober 1987, Seiten 1807-1812, American Chemical Society, Washington, US; L.D. WISE et al.: "1,3-dialkyl-4-(iminoarylmethyl)-1H-pyrazol-5-ols. A series of novel potential antipsychotic agents"<br>* Der ganze Artikel *<br>-- | | RECHERCHIERTE SACHGEBIETE (Int. Cl.4) |
| A | JOURNAL OF MEDICINAL CHEMISTRY, Band 27, Nr. 11, November 1984, Seiten 1396-1400, American Chemical Society, Washington, US; D.E. BUTLER et al.: "(1,3-dialkyl-5-amino-1H-pyrazol-4-yl)arylmethanones. A series of novel central nervous system depressants"<br>* Der ganze Artikel *<br>-- | | |
| A | JOURNAL OF THE CHEMICAL SOCIETY, Band JCPRB4, Nr. 15, 1. Januar 1974, Seiten 1753-1892, Letchworth, GB; T. NISHIWAKI et al.: "Studies on heterocyclic chemistry. Part XIX. Synthesis of 4-aroyl-1-arylpyrazoles from alpha-aroyl-beta-anilinoacrylonitriles and photochemistry of 4-carbonyl-substituted pyrazoles"<br>* Der ganze Artikel *<br>-- ./. | | |

Europäisches Patentamt

# EUROPÄISCHER TEILRECHERCHENBERICHT

Nummer der Anmeldung

EP 90 25 0189
-4-

| EINSCHLÄGIGE DOKUMENTE | | | KLASSIFIKATION DER ANMELDUNG (Int. Cl.4) |
|---|---|---|---|
| Kategorie | Kennzeichnung des Dokuments mit Angabe, soweit erforderlich, der maßgeblichen Teile | betrifft Anspruch | |
| A | JOURNAL OF ORGANIC CHEMISTRY, Band 36, Nr. 17, 27. August 1971, Seiten 2542-2547, Easton, US; D.E. BUTLER et al.: "New general methods for the substitution of 5-chloropyrazoles. The synthesis of 1,3-dialkyl-5-chloropyrazol-4-yl aryl ketones and new 1,3-dialkyl-2-pyrazolin-5-ones"<br>* Der ganze Artikel *<br>-- | | |
| A | JOURNAL OF THE CHEMICAL SOCIETY (C), 1967, Seiten 1494-1497, Letchworth, GB; I.L. FINAR et al.: "Preparation and properties of some pyrazolyl ketones"<br>* Der ganze Artikel *<br>-- | | RECHERCHIERTE SACHGEBIETE (Int. Cl.4) |
| A | US-A-4 008 200 (L. AVAR)<br>-- | | |
| P,A | CHEMICAL ABSTRACTS, Band 111, Nr. 9, 28. August 1989, Seite 754, Zusammenfassung Nr. 78000e, Columbus, Ohio, US; & JP-A-63 316 704 (NISSAN CHEMICAL INDUSTRIES, LTD) 26-12-1988<br>* Zusammenfassung *<br>---- | | |

EPA Form 1505.3 06.78

EP 90 25 0189 -C-

Die Zahl, der in Patentanspruch 1 genannten substituierten 4-Heteroaroylpyrazolen ist so gross, dass eine vollständige Recherche aus ökonomischen Gründen nicht möglich ist (Siehe Richtlinien für die Prüfung im Europäischen Patentamt, Teil B, Kapitel III, 2, Umfang der Recherche). Die Recherche beschränkt sich deshalb ausschliesslich auf diejenigen Endprodukte, die durch physikalische oder chemische Daten charakterisiert sind, d.h. die Beispielsubstanzen.